Europäisches Patentamt

European Patent Office

Office européen des brevets

(19)

(11) Publication number: **0 136 764**
**A2**

(12) **EUROPEAN PATENT APPLICATION**

(21) Application number: **84201421.9**

(22) Date of filing: **05.10.84**

(51) Int. Cl.⁴: **C 07 C 126/02**

(30) Priority: **06.10.83 NL 8303424**

(43) Date of publication of application:
**10.04.85 Bulletin 85/15**

(84) Designated Contracting States:
**AT BE DE FR GB IT NL**

(71) Applicant: **UNIE VAN KUNSTMESTFABRIEKEN B.V.**
**Maliebaan 81**
**NL-3581 CG Utrecht(NL)**

(72) Inventor: **van Nassau, Petrus Johannes Marie**
**Burg. Pylsstraat 4**
**NL-6151 HA Munstergeleen(NL)**

(74) Representative: **Roeffen, Wilhelmus Johannes**
**Maria et al,**
**OCTROOIBUREAU DSM Postbus 9**
**NL-6160 MA Geleen(NL)**

(54) **Process for the preparation of urea.**

(57) An improved process for the preparation of urea from ammonia and carbon dioxide at an elevated temperature and pressure in first and second reaction zones, and the removal of non-converted ammonium carbamate from the urea synthesis solution in first and second stripping zones, wherein the first reaction zone is adapted such that the contents thereof are in indirect heat exchange with the contents of the first stripping zone. Ammonia and carbon dioxide are at least in part reacted to form ammonium carbamate and the ammonium carbamate is in part converted to urea in the first reaction zone, and the resulting effluent is introduced into the second reaction zone wherein further urea formation is effected to form a urea synthesis solution containing non-converted ammonium carbamate. A portion of the urea synthesis solution is introduced into the first stripping zone and another portion is introduced into the second stripping zone wherein the respective portions are heated and stripped to decompose at least part of the ammonium carbamate and form a gas mixture containing ammonia and carbon dioxide. The gas mixture thus formed in the second stripping zone is condensed in the first reaction zone in a manner such that the heat of condensation therein produced is exchanged with the urea synthesis solution in the first stripping zone so as to provide at least a portion of the heat required for the ammonium carbamate decomposition.

EP 0 136 764 A2

## PROCESS FOR THE PREPARATION OF UREA

### Background of the Invention

This invention relates to a process for the preparation of urea.

One process for the preparation of urea which has found wide acceptance is described in European Chemical News Urea Supplement of 17th January, 1969, pages 17-20. In that process a urea synthesis solution formed in a reaction zone at high pressure and temperature is subjected to a stripping treatment at the synthesis pressure. In this stripping treatment, the synthesis solution is heated and contacted countercurrently with gaseous carbon dioxide to decompose a major portion of the carbamate contained therein into ammonia and carbon dioxide. These decomposition products are expelled from the solution and, together with a small quantity of water vapor and the carbon dioxide used for stripping, are separated from the remaining urea product stream as a gas mixture. This gas mixture is subsequently partially condensed in a condensation zone to form an aqueous ammonium carbamate solution which, together with the remaining noncondensed gas mixture, is returned to the reaction zone for the formation of a further quantity of urea. The further condensation of this remaining gas mixture in the reactor provides the heat required for the conversion of ammonium carbamate into urea, and no additional heat need be supplied to the reactor from outside.

The heat required for this stripping treatment is obtained by condensation of high-pressure steam on the outside of the tubes of a vertical heat exchanger in which the stripping takes place. According to this publication, about 1000 kg of about 25 bar steam is required per ton of urea. In practice, however, this consumption of high-pressure steam (25 bar) has now been reduced to about 850 kg per ton urea. Moreover, it is possible to partially recover the heat used in the stripping treatment by condensing the gas mixture which has been separated off, but in view of the relatively low temperature at which this condensation takes place, only steam of 3-5 bar can be formed, which often cannot effectively be used either within or outside of the process. For this reason, and particularly in view of the continually increasing energy prices, the consumption of high-pressure steam must be reduced as far as possible and, depending on local conditions, it may be desirable to avoid or strongly reduce the production of surplus low-pressure steam.

Various proposals have already been made to use the heat released in the condensation of ammonia and carbon dioxide into ammonium carbamate to provide or at least supplement the heat requirements of the stripping treatment.

One such proposal is disclosed in British patent application 2,107,704 wherein ammonium carbamate and urea formation take place in the shell side of a vertical tubular heat exchanger which serves as a reaction zone, and the urea formation continues in an after-reaction zone. The pressure in the shell side of the vertical tubular heat exchanger is preferably about 20 to 120 bar higher than the pressure within the tubes. At least a portion of the urea synthesis solution formed in the after-reaction zone is subjected to a stripping treatment in the tubes of the vertical tubular heat exchanger, and the heat required for this stripping is supplied by a portion of the heat of carbamate formation released in the shell side. By having part of the urea formation take place in the shell side reaction zone, the average temperature there is substantially increased, as a result of which the heat of reaction becomes available at such a high temperature level that an acceptable stripping efficiency can be achieved without the necessity of high-pressure steam.

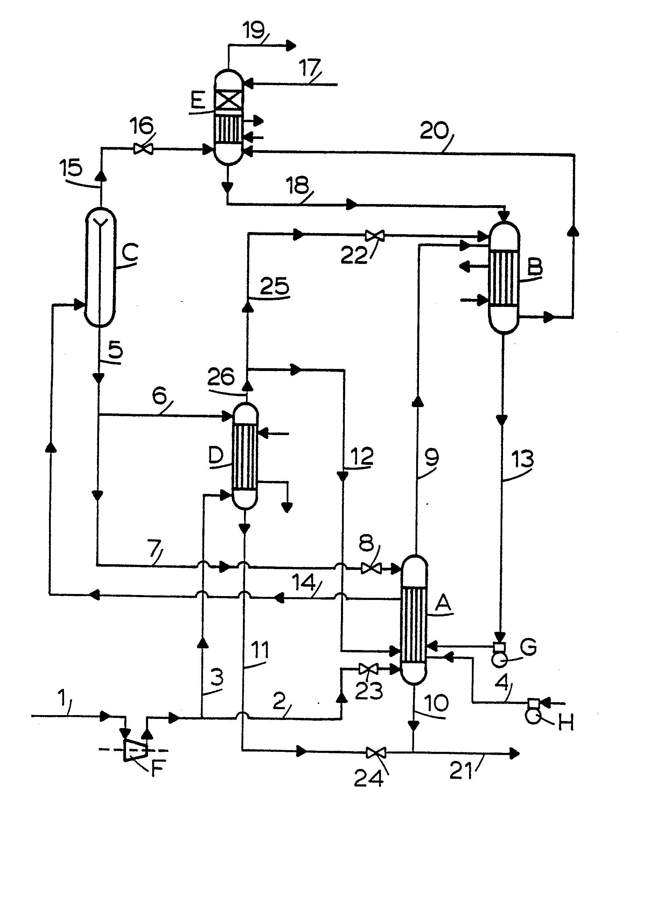

In the embodiment shown in Figure 4 of that patent, only a portion of the urea synthesis solution produced in the after-reaction zone is stripped using the heat of carbamate formation, without the use of an external stripping gas, and the remainder is treated in a heater-decomposer using steam. In this embodiment, the ammonia and carbon dioxide containing off-gas obtained in both stages is condensed in a carbamate condenser wherein the heat of condensation is utilized to make low pressure steam.

A significant concern with this known embodiment is that for the decomposition efficiency to be sufficienctly high, the heater-decomposer must be operated at such a high temperature level that costly construction materials must be used to avoid unacceptable corrosion in the equipment. Moreover, at this higher temperature level there will be an undesirable increase in biuret formation and urea hydrolysis. Therefore, as a practical matter and for safety considerations, lower temperatures will be applied and a lower decomposition efficiency will be accepted, so that the usual stainless steel construction materials can be utilized. However, this lower decomposition efficiency inevitably results in the release of substantial amounts of ammonia and carbon dioxide at lower pressures in the further downstream processing of the stripped urea synthesis solution, which ammonia and carbon dioxide must be returned to the synthesis zone as an aqueous ammonium carbamate solution. The presence of water in the urea synthesis zone has a negative effect on the synthesis efficiency, making it necessary to minimize the amount of water present in this carbamate solution recycled to the synthesis zone as much as possible. Furthermore, in this known process, the gas mixture formed in the heater-decomposer is condensed in a condensation zone which yields low-pressure steam for which, as previously noted, there is limited use in the process.

## Summary of the Invention

The object of the present invention is to provide a process in which the amount of high-pressure steam required for the decomposition of non-converted ammonium carbamate contained in the urea synthesis solution is substantially reduced, without encountering the disadvantages of the above-mentioned known process. According to this invention, this objective is achieved by treating a portion of the urea synthesis solution in a decomposition zone and utilizing the heat released in the condensation of the resulting ammonia and carbon dioxide gases for stripping the remainder of the urea synthesis solution in a stripping zone which exchanges heat with the reaction zone in which this condensation is effected.

The process according to the invention, therefore, relates to the preparation of urea from ammonia and carbon dioxide in which the reaction between ammonia and carbon dioxide is at least in part effected in a first reaction zone which is adapted to exchange heat with a first stripping zone, while further carbamate and urea formation is effected in a second, after-reaction zone to produce a urea synthesis solution. A portion of this urea synthesis solution from the after-reaction zone is treated in the first stripping zone and the remainder is treated in a second stripping zone, to remove non-converted ammonium carbamate and excess ammonia, and the stripped urea synthesis solution thus obtained is processed into product urea, such as a urea solution or solid urea. A particularly improved heat efficiency and utilization of steam can be achieved, in accordance with the invention, by condensing the ammonia and carbon dioxide containing gas mixture formed in the second stripping zone into an ammonium carbamate solution in the first reaction zone in a manner such that the heat of condensation is indirectly exchanged with the urea synthesis solution in the first stripping zone. The term indirect heat exchange as used herein should be understood to mean the transfer of heat between two components across a heat exchange surface, such as the wall of a heat exchanger tube, rather than by direct mixing of the two components, on the one hand, or via the intermediate production of steam, on the other.

In order to maximize the amount of heat that can be transferred from the first reaction zone to the first stripping zone, it is desirable to cause a portion of the ammonium carbamate to convert into urea in this reaction zone. The formation of urea and water in addition to the condensation of ammonia and carbon dioxide causes the temperature in the reaction zone to increase. By providing a sufficiently long residence time, a portion of the ammonium carbamate formed in the first reaction zone will be converted to urea, resulting in an increased temperature and significantly greater transfer of heat to the first stripping zone. Depending on the pressure applied in the reaction zone, this temperature increase may amount to between about 10 to 20°C as compared to the circumstance where urea formation does not take place. Preferably, the amount of urea formed in the first reaction zone will be between about 70 and 90 percent of the equilibrium amount of urea that would be formed under the prevailing conditions. To obtain optimum utilization of the heat released in the reaction zone, it is desirable to provide for intensive mixing of the reaction zone contents, which ensures that there is a sufficient temperature difference over the entire heat-exchanging area and promotes heat transfer to the stripping zone.

The gas mixture formed in the second stripping zone is preferably obtained by heating a portion of the urea synthesis solution with high pressure steam while contacting it with a countercurrent flow of a stripping gas comprised of a portion of the fresh carbon dioxide feed required for the urea formation. The resulting carbon dioxide enriched gas mixture is fed to the first reaction zone where it is combined under reaction conditions with a liquid ammonia feed and a recirculating ammonium carbamate solution. The remainder of the carbon dioxide required for the urea formation is supplied as a stripping gas to the first stripping zone. By preference, between about 30 and 50 percent of the amount of carbon dioxide required for the urea formation is introduced initially into the second stripping zone.

The division of the urea synthesis solution from the second, after-reaction zone between the first and second stripping zones may vary, but most favorable results are obtained when the proportion of urea synthesis solution introduced into the second stripping zone is between about 40 and 60 percent of the total amount. Within that range, the heat released in the first reaction zone upon condensation of the gas mixture obtained in the second stripping zone is sufficient to provide the entire heat requirement in the first stripping zone.

In the first stripping zone, a pressure from about 125 to 250 bar can be applied. Mostly, a pressure in the range of between about 175 and 210 bar will be chosen because then the heat is released and transferred at such a level that the stripping operation proceeds at a good efficiency in a short period of time. The higher the pressure in the reaction zone, the higher the temperature at which the condensation of carbon dioxide and ammonia to carbamate takes place, so that more heat becomes available for carbamate decomposition in the stripping zone. Preferably, a pressure differential of at least about 20 bar between the first reaction zone and the first stripping zone is chosen to provide a large enough temperature differential to ensure an adequate driving force for sufficient heat exchange from the reaction zone to the stripping zone. A pressure differential of more than about 120 bar is theoretically possible but requires a complex and costly design of the equipment accommodating the reaction and stripping zones. Most preferably, the pressure chosen for the first reaction zone should be in the range of between about 20 and 60 bar higher than the pressure in the first stripping zone.

## Brief Description of the Drawing

The invention will now be elucidated with reference to the figure, illustrating a method embodying the present invention.

## Description of the Preferred Embodiment

In the figure, A denotes a reactor-stripper in which the first reaction zone is in the shell side and the first stripping zone in the tubes of a vertical heat exchanger. B denotes a carbamate condenser, C an after-reactor, D a stripper, and E a scrubber. F, G, and H denote, respectively, a carbon dioxide compressor, a carbamate pump, and a ammonia pump.

The reaction zone of reactor-stripper A, after-reactor C, and stripper D, are preferably all maintained at the same pressure. Likewise, it is preferred to have the same pressure maintained in the stripping zone of reactor-stripper A, in carbamate condenser B, and in scrubber E, which for instance must be about 20 to 60 bar lower than the pressure maintained in units A, C, and D.

A urea synthesis solution is obtained in after-reactor C at a pressure of, for instance, about 175 to 210 bar, and contains, in addition to urea and water, non-converted carbamate and free ammonia. This urea synthesis solution is discharged through line 5 and split into two portions. The first portion is passed through line 6 into stripper D, where it is subjected to a stripping treatment at the same pressure as in after-reactor C. This stripping treatment is effected counter-current to gaseous carbon dioxide while heat is supplied by high-pressure steam. Carbon dioxide is supplied to this stripper through lines 1 and 3 and brought up to the required pressure of 175 to 210 bar by compressor F. Air or another oxygen-containing gas mixture is added to the carbon dioxide to keep the materials of construction coming into contact at high temperature with ammonium carbamate-containing solutions in a passive condition. The amount of carbon dioxide used in the stripping treatment in stripper D is preferably between about 30 and 50 percent of the total amount of fresh carbon dioxide required in the process.

After this stripping treatment, a stripped urea synthesis solution is discharged from stripper D through line 11, expansion valve 24 and line 21, and is treated in a way known per se for the removal of carbamate still present. A gas mixture is discharged from stripper D through line 26 which consists of the fresh carbon dioxide supplied, and the ammonia, carbon dioxide, and water expelled from the urea synthesis solution by the stripping treatment. This gas mixture is passed through line 12 to the reaction zone of reactor-stripper A. If desired, a small portion of the gas mixture discharged through line 26 can be fed to carbamate condenser B through line 25 and expansion valve 22.

In addition to the gas mixture from stripper D, the reaction zone of reactor-stripper A is fed with the ammonia required in the process (which is supplied through line 4 by pump H) and with the carbamate solution formed in carbamate condensor B (which is pumped into this reaction zone through line 13 and pump G). In the reaction zone of reactor-stripper A, a major portion of the ammonia and carbon dioxide supplied through lines 12 and 4 is converted into ammonium carbamate, and this carbamate, together with the carbamate in the carbamate solution supplied through line 13, are in part converted into urea and water.

Heat is released in the formation of ammonium carbamate whereas heat is consumed in the conversion of carbamate into urea and water. There is, however, a net heat surplus which is used for the decomposition of non-converted carbamate in the stripping zone. Since the gas mixture fed to the first reaction zone through line 12 contains at least a major part of the carbon dioxide that was fed into stripper D, and since the carbamate and urea formation are effected in this reaction zone at a higher pressure than that prevailing in the stripping zone of reactor-stripper A, enough heat becomes available at a relatively high temperature level to decompose a very substantial portion of the carbamate present in the solution supplied to this first stripping zone without the need for heat from an external source. By continuing the conversion of carbamate into urea and water in the first reaction zone until a substantial part, for instance more than half, of the equilibrium amount of urea achievable under the reaction conditions applied has been formed, a further increase in temperature is accomplished and the portion of the surplus heat transferred to the stripping zone becomes larger. This effect will be larger as equilibrium is approached. In general, therefore, the amount of urea formed will preferably be more than about 70 percent of the equilibrium value. However, urea formation in excess of 90 percent of the equilibrium amount necessitates substantially longer residence times because of the strongly decreased reaction rate, and thus requires an unattractively large volume in the reaction zone.

The first reaction zone preferably is intensively mixed so as to achieve optimum uniformity in the temperature distribution and optimum heat transfer. To a certain extent, this is already accomplished by having the gas mixture and the carbamate solution flow upwards through the reaction zone. Mixing, and thus heat transfer, is further improved by installation of baffles, guide plates, or similar elements.

The effluent from this first reaction zone, consisting of a solution of urea, water, ammonia, and ammonium carbamate and a gas mixture consisting of ammonia, carbon dioxide, water, and inerts, is passed through line 14 to after-reactor C, where urea formation is continued until at least about 90 percent of the equilibrium amount of urea achievable under the reaction conditions applied here has been formed. The required heat in after-reactor C is obtained by permitting part of the gas mixture supplied to the reaction zone of reactor-stripper A through 12 to remain uncondensed and to pass as a gas through line 14 into the after-reaction zone where it is condensed, releasing both the heat of condensation and heat of carbamate formation.

The remainder of the urea synthesis solution formed in after-reactor C (i.e., that portion not sent to stripper D) flows through line 7 and, after reduction of the pressure to the pressure in the first stripping zone (for instance 140 bar) via expansion valve 8, is introduced into the space above the tubes of reactor-stripper A. Here the gas mixture consisting of ammonia, carbon dioxide, and water that is released on the pressure decrease is separated from the remaining urea synthesis solution. The solution flows downward through the stripping zone along the internal walls of the tubes countercurrent to a stripping gas containing between about 50 and 70 percent of the amount of carbon dioxide required in the synthesis, which is supplied through line 2 and expansion valve 23. The urea synthesis solution flowing downward through the tubes is heated by heat exchange with the reaction zone and stripped with the carbon dioxide, and the gas mixture thus formed, together with the stripping gas supplied, flows upwardly to the space above the tubes and is discharged through line 9, together with the gas mixture released on the pressure decrease, and led into carbamate condenser B. The stripped urea synthesis solution is discharged from the bottom of reactor-stripper A through line 10 and is combined in line 21 with the stripped urea synthesis solution discharged from stripper D through line 11 for further processing.

In carbamate condenser B, a major portion of the gas mixture fed from the first stripping zone via line 9 is condensed by heat-exchange with water, which is thereby converted into low-pressure steam. However, the pressure of the steam formed in the carbamate condenser is only 3 to 5 bar, and there is often little use for this low pressure steam either in or outside of the process. The formation of this low pressure steam can be avoided, however, if the heat of condensation released in carbamate condenser B is exchanged with the stripped urea synthesis solution in line 21 after the latter has been expanded to a pressure of, for instance 15 to 25 bar. Thus, according to this embodiment, the pressure of the stripped urea solutions obtained in stripper D and reactor-stripper A is preferably reduced to about 15 to 25 bar, and a further amount of ammonium carbamate still present in the stripped solution is decomposed by passing it through carbamate condenser B. This further decomposition is effected by indirect heat exchange with the gas mixture condensing at the pressure of the stripping zone of reactor-stripper A that is obtained in the stripping treatment in reactor-stripper A.

-11-

The carbamate solution formed by the condensation in carbamate condenser B is discharged through line 13, brought up to the pressure of the reaction zone of reactor-stripper A by pump G and pumped into said zone. The portion of the gas mixture that has not been condensed in carbamate condenser B, consisting mainly of inerts introduced with the carbon dioxide, but still containing ammonia, carbon dioxide, and water, is introduced into scrubber E through line 20.

A gas mixture containing inert gases and equilibrium amounts of ammonia, carbon dioxide, and water also remains uncondensed in after-reactor C. This gas mixture is discharged through line 15 and expansion valve 16 and passed into scrubber E where ammonia and carbon dioxide are recovered by scrubbing with water or dilute carbamate solution supplied through line 17, and the heat of absorption is discharged. Scrubber E is preferably maintained at the same pressure as the stripping zone of reactor-stripper A and carbamate condenser B. The remaining non-absorbed off-gas mixture is discharged from the system through line 19, and the relatively dilute carbamate solution obtained in scrubber E is led to carbamate condenser B through line 18.

## Example

Using the process described, urea is prepared according to the embodiment represented in the figure in a plant having a production capacity of 1500 ton of urea per day. All amounts are given in kg per hour. The high pressure part of the plant was made of the usual stainless steel construction material.

The reaction zone of reactor-stripper A, after-reactor C, and stripper D are all maintained at a pressure of about 186.3 bar, and carbamate condenser B, scrubber E, and the stripping zone of reactor-stripper A are maintained at a pressure of about 137.3 bar.

Through $NH_3$ pump H, 34,717 kg $NH_3$ of 40°C is fed to the first reaction zone of reactor-stripper A, and via $CO_2$ compressor F, 45,832 kg of $CO_2$ and 1,761 kg inert gases, mainly air, of 120°C is fed to the plant. This first reaction zone is further supplied via pump G with 98,017 kg carbamate solution consisting of 41,519 kg $NH_3$, 46,108 kg $CO_2$, and 10,390 kg $H_2O$, and through 12 with a gas mixture, originating from stripper D, consisting of 23,441 kg $NH_3$, 27,228 kg $CO_2$, 1,939 kg $H_2O$, and 764 kg inert gases. The volume of the reaction zone, and thus the residence time, is chosen so that at the pressure of about 186.3 bar and the corresponding temperature of about 185°C, a gas-liquid mixture is formed of which the liquid phase is composed of 60,626 kg $NH_3$, 28,970 kg $CO_2$, 52,086 kg urea, and 27,550 kg $H_2O$, while the gas phase consists of 9,536 kg $NH_3$, 6,169 kg $CO_2$, 405 kg $H_2O$, and 764 kg inert components.

This gas-liquid mixture is introduced into after-reactor C, via line 14, wherein a urea synthesis solution is formed, consisting of 65,108 kg urea, 58,718 kg $NH_3$, 22,960 kg $CO_2$, and 31,680 kg $H_2O$. Half of this urea synthesis solution is treated in stripper D countercurrent to 22,446 kg $CO_2$, while the second half, after reduction of the pressure to 137.3 bar, is treated in the stripping zone of reactor-stripper A countercurrent to 23,386 kg $CO_2$.

In stripper D, which is heated with high-pressure steam of 22.6 bar, the larger part of the carbamate present in the solution supplied through line 6 is decomposed and the $NH_3$ and $CO_2$ released are expelled, so that through line 26, 60,199 kg gas mixture, consisting of 26,439 kg $NH_3$, 30,711 kg $CO_2$, 2,187 kg $H_2O$, and 862 kg inert gases, is discharged. Of this gas mixture, an amount of 53,372 kg is passed through 12 to the reaction zone of reactor-stripper A, while the remainder is sent to carbamate condenser B after expansion to 137.3 bar. From stripper D a urea solution is discharged which, besided 31,098 kg urea and 13,214 kg $H_2O$, contains 3,750 kg $NH_3$ and 4,289 kg $CO_2$.

In the stripping zone of reactor-stripper A, likewise a major portion of the carbamate present in the solution supplied through line 7 is decomposed, thereby releaseing gaseous $NH_3$ and $CO_2$, which are removed via line 9 together with the gaseous $CO_2$ supplied. The gas mixture thus released contains 20,821 kg $NH_3$, 24,436 kg $CO_2$, 1,660 kg $H_2O$, and 899 kg inert gases. A major portion of these gases is condensed in carbamate condenser B and the carbamate solution obtained is pumped into the reaction zone of reactor stripper A. The urea solution discharged from the stripping zone of reactor-stripper A contains 32,391 kg urea, 14,131 kg $H_2O$, 8,630 kg $NH_3$, and 10,550 kg $CO_2$.

The heat required in the stripping zone of reactor-stripper A is entirely supplied by the heat released in the reaction zone. In stripper D, 23,461 kg high-pressure steam of 22.6 bar is required for the stripping treatment. In carbamate condenser B, 26,884 kg low-pressure steam of 4.4 bar is obtained.

## WHAT IS CLAIMED IS:

1. In a process for the preparation of urea from ammonia and carbon dioxide, having first and second reaction zones and first and second stripping zones wherein said first reaction zone is adapted such that the contents thereof are in indirect heat exchange with the contents of said first stripping zone, and which process comprises the steps of:

reacting at least a part of said ammonia and carbon dioxide to form ammonium carbamate and converting a part of said ammonium carbamate to urea in said first reaction zone, introducing effluent from said first reaction zone into said second reaction zone wherein further urea formation is effected to form a urea synthesis solution containing urea, non-converted ammonium carbamate and free ammonia;

introducing a portion of said urea synthesis solution from said second reaction zone into said first stripping zone wherein said solution is heated and stripped, thereby decomposing at least a portion of said non-converted ammonium carbamate, and separating the gaseous mixture thus formed, containing ammonia and carbon dioxide, from the remaining stripped urea synthesis solution;

introducing a further portion of said urea synthesis solution from said second reaction zone into said second stripping zone wherein said solution is heated and stripped, thereby decomposing at least a portion of said non-converted ammonium carbamate, and separating the gaseous mixture thus formed, containing ammonia and carbon dioxide, from the remaining stripped urea synthesis solution; and

thereafter further processing the stripped urea solutions from said first and second stripping zones into product urea,

the improvement essentially comprising condensing, in said first reaction zone, gaseous mixture containing ammonia and carbon dioxide formed in said second stripping zone such that the heat of condensation thereby produced in said first reaction zone is exchanged with the urea synthesis solution in said first stripping zone so as to provide at least a portion of the heat required for the decomposition of ammonium carbamate contained in said solution.

2. The improved process according to claim 1, wherein the gas mixture formed in the second stripping zone is condensed and at least in part converted into a urea-containing ammonium carbamate solution in said first reaction zone.

3. The improved process according to claim 1, wherein at least a portion of the carbon dioxide required for the urea preparation is fed to the second stripping zone as a stripping agent, thereby enriching the gas mixture formed therein with respect to carbon dioxide.

4. The improved process according to claim 3, wherein said carbon dioxide enriched gas mixture formed in the second stripping zone, as well as ammonia, is supplied to said first reaction zone.

5. The improved. process according to claim 3, wherein a portion of the carbon dioxide required for the urea preparation is fed to the first stripping zone.

6. The improved process according to claim 3, wherein between about 30 and 50 percent of the carbon dioxide required for the urea preparation is fed to the second stripping zone.

7. The improved process according to claim 1, wherein between about 40 and 60 percent of the urea synthesis solution formed in said second reaction zone is fed to said second stripping zone.

8. The improved process according to claim 1, wherein said stripped urea solutions contain further amounts of ammonium carbamate which are decomposed by passing these solutions, after their pressure has been reduced to between about 15 and 25 bar, in indirect heat exchange with the gas mixture obtained in said first stripping zone such that the heat of condensation of said first stripping zone gas mixture provides heat to decompose ammonium carbamate contained in said stripped urea solutions.

1/1